# EUROPÄISCHE PATENTANMELDUNG

(11) **EP 2 484 406 A1**
(43) Veröffentlichungstag der Anmeldung: **08.08.2012**
(21) Anmeldenummer: 12150659.6
(22) Anmeldetag: 10.01.2012
(51) Int. Cl.: A61N 1/375

(54) **Elektrodenkabelbinder**

(30) Priorität: 04.02.2011 US 201161439381 P
(71) Anmelder: BIOTRONIK SE & Co. KG, 12359 Berlin (DE)
(72) Erfinder: Kolberg, Gernot, 12161 Berlin (DE); Dörr, Thomas, 12437 Berlin (DE)
(74) Vertreter: Lindner-Vogt, Karin L.

(57) **Zusammenfassung**

Gemäß eines ersten Aspektes wird eine Implantierbare Vorrichtung zur reversiblen Aufnahme eines Elektrodenleitungsabschnittes mindestens einer Elektrodenleitung beschreiben, welche eine erste Außenfläche, und eine zweite Außenfläche, die sich beabstandet parallel zur ersten Außenfläche erstreckt, umfasst, wobei die erste Außenfläche mindestens ein Führungsmittel umfasst, mit dessen Hilfe der Elektrodenleitungsabschnitt geführt und aufgenommen werden kann. Gemäß eines zweiten Aspektes wird ein elektromedizinisches Implantat beschreiben, bei dem die implantierbare Vorrichtung in das Gehäuse integriert dies. Das elektromedizinische Implantat umfasst dabei neben der soeben beschriebenen Vorrichtung eine elektronische Schaltung mit Energieversorgung, ein mehrteiliges Gehäuse, welches die Schaltung und die Energieversorgung hermetisch dicht umschließt, und ein Anschlussgehäuse, welches am mehrteiligen, hermetisch dichten Gehäuse befestigt ist und welches Anschlüsse für mindestens Elektrodenleitung aufweist, wobei die Anschlüsse mit der elektronischen Schaltung elektrisch verbunden sind. Das elektromedizinische Implantat zeichnet sich dabei dadurch aus, dass die zweite Außenfläche der Vorrichtung einen Teil des Gehäuses bildet.

## Beschreibung

Die Erfindung betrifft eine implantierbare Vorrichtung zur reversiblen Aufnahme eines Elektrodenleitungsabschnittes mindestens einer Elektrodenleitung, um diesen Abschnitt der mindestens einen Elektrodenleitung in einer Gewebs- oder Implantatstasche, in der ein elektromedizinisches Implantat implantiert ist, zu verstauen.

Elektromedizinische Implantate umfassen in der Regel
- eine elektronische Schaltung mit Energieversorgung,
- ein mehrteiliges Gehäuse, welches die Schaltung und die Energieversorgung hermetisch dicht umschließt, und
- ein Anschlussgehäuse, welches am mehrteiligen, hermetisch dichten Gehäuse befestigt ist und welches Anschlüsse für mindestens Elektrodenleitung aufweist, wobei die Anschlüsse mit der elektronischen Schaltung elektrisch verbunden sind.

Solche Implantate werden in der Regel dazu eingesetzt, an bestimmten Stellen des Körpers eine therapeutische Wirkung zu erzielen, nachdem beispielsweise über eine diagnostische Funktion im elektromedizinischen Implantat der Bedarf für diese Therapie festgestellt wurde. Bei den elektromedizinischen Implantaten kann es sich nicht abschließend um Herzschrittmacher, implantierbare Defibrillatoren oder Cardioverter, Nervenstimulatoren oder Hirnstimulatoren handeln. In letzter Zeit wurden solche elektromedizinischen Implantate aber auch zur Aufnahme von körpereigenen Signalen genutzt, welche in dem Implantat gesammelt werden und entweder ausgewertet werden oder aber zur Auswertung an ein externes Gerät weitergeleitet werden können. Diese Technik der so genannten Telemetrie hat sich bei allen elektromedizinischen Implantaten durchgesetzt, weshalb auch die übrigen genannten Implantate mit einer solchen Funktionalität versehen werden kann.

Wie erwähnt umfassen die elektromedizinischen Implantate ein Anschlussgehäuse, welches am mehrteiligen, hermetisch dichten Gehäuse befestigt ist und welches Anschlüsse für mindestens eine Elektrodenleitung aufweist. Eine solche Elektrodenleitung besteht aus einem lang gestreckten Körper, der von außen aus einem isolierenden Material wie Silikon oder Polyurehtan besteht. Der Körper weist ein proximales und ein distales Ende auf. Am proximalen Ende befindet sich mindestens eine Steckverbindung welche mit dem Anschluss im Anschlussgehäuse - in der Regel eine Steckeraufnahme - verbunden werden kann. Der Stecker ist genormt und kann gemäß einer der Normen IS-1, IS-4 oder DF-1 gestaltet sein. Jede der elektrisch aktiven Kontakte des Steckers ist mit einer Verbindungsleitung elektrisch verbunden, welche in der Regel je einer elektrisch aktiven Fläche am oder in der Nähe des distalen Endes liegt. Jede dieser Verbindungsleitungen ist isoliert geführt. Die elektrisch aktiven Flächen - kurz auch "Elektrode" genannt - dienen dazu, an der zu behandelnden Körperpartie wie beispielsweise im oder am Herz, im oder am Gehirn oder an Nerven) eine elektrische Therapie zu induzieren oder aber Messsignale zur Diagnostik aufzunehmen.

Elektrodenleitungen werden in der Regel in verschiedenen Längen angeboten. Beispielsweise werden von Herzelektrodenleitungen drei diskrete Längen angeboten. Der implantierende Arzt hat also die für den Patienten individuell geeignete Länge abzuschätzen und dann die Elektrodenleitungslänge auszuwählen, die möglichst dicht über der geschätzten Länge liegt. Da nur drei Längen zur Verfügung stehen, ist die Elektrodenleitung mehr oder weniger zu lang. Da der Arzt aber nicht die Möglichkeit hat, die Elektrodenleitung zu kürzen und dadurch individuell an den Patienten anzupassen, wickelt er den überschüssigen Elektrodenleitungsabschnitt am proximalen Ende der Elektrodenleitung unmittelbar distal des Steckers um das elektromedizinische Implantat und verstaut sie so mit dem Gerät in der dazugehörigen Gewebetasche.

Diese Vorgehensweise hat einige Nachteile, die durchaus die sichere Funktion des elektromedizinischen Implantats und der damit verbundenen Diagnostik und/oder Therapie stören können, was zu lebensbedrohlichen Zuständen für den Patienten führen kann.

Ein wichtiger Nachteil ist, dass sowohl das elektromedizinische Implantat als auch die umwickelten Elektrodenleitungsabschnitte in kürzester Zeit von körpereigenem Gewebe umwachsen ist. Das Gewebe dringt dabei in jeden freien Raum der Gewebstasche und umschließt so den umwickelten Elektrodenleitungsabschnitt.

In diesem Fall muss ein Arzt bei einem Wechsel des elektromedizinischen Implantats, welches beispielsweise aufgrund der begrenzten Energiekapazität das "end of life" erreicht hat, die umwickelten Elektrodenleitungsabschnitte frei präparieren, um das elektromedizinische Gerät freizulegen und austauschen zu können. Dabei besteht das Risiko, dass mit den scharfschneidigen Präparationswerkzeugen die Isolation der Elektrodenleitung verletzt wird und dadurch Verbindungsleitungen freigelegt werden bzw. Kurzschlüsse zwischen den Verbindungsleitungen geschehen. Dies kann zu Fehlmessungen und daraus induzierten Fehltherapien führen, die zu lebensbedrohlichen Zuständen führen können.

Weiterhin kann es bei unvorteilhaftem Verstauen der Elektrodenleitungsabschnitte zu einer ungünstigen Anhäufung der Elektrodenleitungen kommen, die zu einer stellenweise erhöhten Materialanhäufung führt, die für den Patienten störend ist. An diesen Stellen kann außerdem ein punktueller Druck auf das umliegende Gewebe entstehen, was Reizungen verursacht, welche im schlimmsten Fall zu entzündlichen Reaktionen oder zu nekrotischen Zuständen des unmittelbaren Gewebes führt.

Aber auch diese Materialanhäufung kann durch unvorteilhaftes Verstauen der Elektrodenleitungsabschnitte zu einer Schädigung der Elektrodenleitung führen. Sie führt bei Einwachsen zu einem erhöhten Druck auf die einzelnen Verbindungsleitungen, weshalb diese punktuell besonders gequetscht oder gewalkt werden, weil die Verbindungsleitungen nicht ausweichen können. Die Folge können Beschädigungen des lang gestreckten isolierenden Körpers sein. Auch dies kann zu Kurzschlüssen oder Fehldiagnosen/-therapien führen. Nachteilig ist ebenfalls, dass bei unvorteilhafter Verstauung die Elektrodenleitungsabschnitte gegeneinander gerieben werden, was ebenfalls zu Beschädigungen führen kann.

Aus dem Stand der Technik ist keine Lösung dieser Problematik bekannt. Deshalb besteht die Aufgabe darin, eine implantierbare Vorrichtung zu schaffen, welche die genannten Nachteile vermeidet und dem Arzt ein Verstauungshilfsmittel bietet.

Die Aufgabe wird durch die unabhängigen Ansprüche 1 und 13 gelöst.

Gemäß eines ersten Aspektes wird eine Implantierbare Vorrichtung zur reversiblen Aufnahme eines Elektrodenleitungsabschnittes mindestens einer Elektrodenleitung beschrieben, welche eine erste Außenfläche, und eine zweite Außenfläche, die sich beabstandet parallel zur ersten Außenfläche erstreckt, umfasst, wobei die erste Außenfläche mindestens ein Führungsmittel umfasst, mit dessen Hilfe der Elektrodenleitungsabschnitt geführt und aufgenommen werden kann. Vorzugsweise ist der Abstand zwischen den beiden Außenflächen wesentlich geringer als die Ausdehnung der Außenfläche, vorzugsweise ist der Abstand zwischen 0,5 mm und 10mm dimensioniert. So ist sichergestellt, dass die Vorrichtung in einfacher Weise neben dem elektromedizinischen Implantat in der Gewebstasche verstaut werden kann, ohne dass zu viel Platz benötigt wird. Weiterhin wird durch die Dimensionierung der Vorrichtung die Handhabung für den Arzt erleichtert. Er kann die Elektrodenleitungsabschnitte, die er bisher ohne Ordnung in der Gewebstasche verstauen musste, geordnet im Führungsmittel unterbringen und so wesentlich den Austausch des elektromedizinischen Implantates erleichtern.

Das mindestens eine Führungsmittel der implantierbaren Vorrichtung kann in die erste Außenfläche integriert, bevorzugt so eingelassen oder vertieft sein, so dass der mindestens eine Elektrodenleitungsabschnitt nicht die erste Außenfläche überragt. So wird verhindert, dass die Elektrodenleitungsabschnitte einen Druck auf das Gewebe ausüben und so Entzündungen oder nektrotische Zustände hervorrufen.

Gemäß einer ersten Weiterbildung ist das mindestens eine Führungsmittel um eine Achse herum angelegt, welche eine Normale zur ersten Außenfläche ist, so dass der mindestens eine Elektrodenleitungsabschnitt im Führungsmittel um die Achse herum geführt werden kann. Mit anderen Worten besteht das Führungsmittel aus Aussparungen in der ersten Außenfläche, welche eine Dicke aufweisen, die geringer ist als der Abstand zwischen der ersten und der zweiten Außenfläche. Die Führungsmittel sind dabei in einem Bogen angeordnet, der sich um eine imaginäre Achse herum erstreckt, so dass die Elektrodenleitungsabschnitte um die verbleibende erste Außenfläche geführt werden kann. Das kann auch bedeuten, dass die Führungsmittel den größten Abschnitt der Vorrichtung abdecken, während die verbleibende Außenfläche einen geringen Teil der Vorrichtung abdeckt. Durch die Formung als Aussparung steht die übrige Außenfläche nach außen ab, das heißt, bezogen auf die zweite Außenfläche habt das mindestens eine Führungsmittel und die Außenfläche ein voneinander verschiedenes Höhenniveau. Dadurch wird die Führung der Elektrodenleitungsabschnitte noch zusätzlich erleichtert ist.

Bevorzugt sind in dieser ersten Weiterbildung die Führungsmittel so geformt, dass es die erste Außenfläche kreis-, gleichdick-, oval- oder achteckförmig mit abgerundeten Ecken umschließt, so dass ein zylinderartiges Gebilde entsteht. Das zylinderartige Gebilde entsteht dabei dadurch, dass das mindestens eine Führungsmittel als Aussparung geformt ist und sich um die imaginäre Achse herum erstreckt. Dadurch werden Beschädigungen des Elektrodenleitungsabschnittes vermieden, da keine Kanten oder Ecken vorliegen, an denen das isolierende Material des lang gestreckten Körpers des Elektrodenleitungsabschnittes beschädigt werden kann.

Weiter bevorzugt umfasst das Führungsmittel in Zusammenhang mit dieser Weiterbildung der implantierbaren Vorrichtung Aushöhlungen, die die erste Außenfläche zumindest teilweise seitlich unterhöhlt, um so ein Verrutschen des mindestens einen Elektrodenleitungsabschnittes zu verhindern. Dadurch entstehen über dem Führungsmittel befindliche Abschnitte der ersten Außenfläche, die verhindern, dass die Elektrodenleitungsabschnitte aus den Führungsmitteln herausrutschen und so zu höherem Materialauftrag führen.

Die implantierbare Vorrichtung gemäß der ersten Weiterbildung umfasst in einer Ausführungsform zusätzlich eine umlaufende Außenkante, die die Vorrichtung begrenzt, wobei die umlaufende Außenkante an die Außenkontur eines elektromedizinisches Implantats angepasst ist. So ist das Einpassen in die Gewebetasche vereinfacht und der Platzbedarf optimiert. Denkbar ist dabei auch, dass die implantierbare Vorrichtung mit dem elektromedizinischen Implantat reversibel anbringbar ist, um so im Gebrauch ein Verrutschen der Vorrichtung zum elektromedizinischen Implantat zu verhindern und gleichzeitig das Lösen beim Austausch des elektromedizinischen Implantates zu ermöglichen. Solche Anbringungsmöglichkeiten können beispielsweise durch Clips an der zweiten Außenfläche erfolgen, durch welche die Vorrichtung an das elektromedizinische Implantat geclipst werden können. Eine andere Anbringungsmöglichkeit wäre beispielsweise das Anbringen eines lösbaren Klebemittels an der zweiten Außenfläche, durch die die Vorrichtung an das elektromedizinische Implantat geklickt werden kann.

Gemäß einer zweiten Weiterbildung der implantierbaren Vorrichtung ist das mindestens eine Führungsmittel als vorgeformte Bahn in die erste Außenfläche integriert, bevorzugt eingelassen oder vertieft ist, in welcher der mindestens eine Elektrodenleitungsabschnitt aufgenommen werden kann. Dadurch entsteht eine lang gestreckte Mulde in der ersten Außenoberfläche, welche so dimensioniert ist, dass der mindestens eine Elektrodenleitungsabschnitt so geführt ist, dass er nicht oder kaum über der ersten Außenfläche herausragt. Auch in dieser Weiterbildung kann die erste Außenfläche zumindest teilweise seitlich unterhöhlt sein, um so ein Verrutschen des mindestens einen Elektrodenleitungsabschnittes zu verhindern.

Vorzugsweise ist die vorgeformte Bahn der zweiten Weiterbildung so gestaltet und in die erste Außenfläche integriert, bevorzugt eingelassen oder vertieft ist, dass sie an jeder Stelle der Außenfläche nur einmal geführt wird. Dadurch wird verhindert, dass die mindestens einen Elektrodenleitungsabschnitte übereinander liegen. Alternativ ist die vorgeformte Bahn so gestaltet und in die erste Außenfläche integriert, bevorzugt eingelassen oder vertieft ist, dass die Bahn Aussparungen an Stellen aufweist, an denen der mindestens eine Elektrodenleitungsabschnitt mehrfach übereinander verläuft. So werden entzündliche oder nekrotische Zustandsänderungen des umliegenden Gewebes vermieden, da die Elektrodenleitungsabschnitte nicht über die erste Außenfläche ragt. Ebenso wird das Gegeneinanderreiben der Elektrodenleitungsabschnitte verhindert.

Auch die implantierbare Vorrichtung gemäß der zweiten Weiterbildung umfasst in einer Ausführungsform zusätzlich eine umlaufende Außenkante, die die Vorrichtung begrenzt, wobei die umlaufende Außenkante an die Außenkontur eines elektromedizinisches Implantats angepasst ist. So ist das Einpassen in die Gewebetasche vereinfacht und der Platzbedarf optimiert. Denkbar ist dabei auch, dass die implantierbare Vorrichtung mit dem elektromedizinischen Implantat reversibel anbringbar ist, um so im Gebrauch ein Verrutschen der Vorrichtung zum elektromedizinischen Implantat zu verhindern und gleichzeitig das Lösen beim Austausch des elektromedizinischen Implantates zu ermöglichen. Solche Anbringungsmöglichkeiten können beispielsweise durch Clips an der zweiten Außenfläche erfolgen, durch welche die Vorrichtung an das elektromedizinische Implantat geclipst werden können. Eine andere Anbringungsmöglichkeit wäre beispielsweise das Anbringen eines lösbaren Klebemittels an der zweiten Außenfläche, durch die die Vorrichtung an das elektromedizinische Implantat geklickt werden kann.

Sowohl die erste als auch die zweite Weiterbildung der implantierbaren Vorrichtung kann bevorzugt aus einem harten, nicht formbaren Material oder weichen, verformbaren Material gefertigt sein. Beispielsweise kann es sich dabei um Polypropylen, Polyethylen, Polyvinylchlorid, Polymethylmethacrylat, Polymethylmethacrylat, Polytetrafluorethylen, Polyvinylalkohol, Polyurethan, Polybuthylenterephthalat, Silikone, Polyphosphazen sowie deren Copolymere und Blends handeln, bevorzugt aber um Epoxydharz oder Polypropylen als harte Materialien und beispielsweise Silikon als weiches Material handeln. Besonders bevorzugt ist das harte oder weiche Material biodegradierbar oder bioresorbierbar, wobei das biodegradierbare oder bioresorbierbare Material besonders bevorzugt eine biodegradierbare Metalllegierung wie beispielsweise eine Magnesium- oder Eisenlegierung, ein biodegradierbares Polymer wie beispielsweise Polydioxanon, Polyglycolid, Polycaprolacton, Polylactide (Poly-L-Lactid, Poly D,L Lactid, und Copolymere sowie Blends wie Poly(L-Lactid-co-glycolid), Poly(D,L-lactid-co-glycolid), Poly(L-Lactid-co-D,L-Lactid), Poly(1-Lactid-co-trimethylen carbonat, Triblockcopolymere), Polysaccharide (Chitosan, Levan, Hyaluronsäure, Heparin, Dextran, Cellulose etc.), Polyhydroxyvalerat, Polyhydroxibuttersäure, Ethylvinylacetat, Polyethylenoxid, Polyphosphorylcholin, Fibrin, Albumin oder Silikon ist.

Zusätzlich kann die Vorrichtung gemäß beider Weiterbildungen mit einer oder mehreren pharmazeutisch aktiven Substanzen versehen sein. Eine "pharmazeutisch aktive Substanz" im Sinne der Erfindung ist ein pflanzlicher, tierischer oder synthetisierter Wirkstoff, der in geeigneter Dosierung als Therapeutika zur Beeinflussung von Zuständen oder Funktionen des Körpers, als Ersatz für natürlich vom menschlichen oder tierischen Körper erzeugte Wirkstoffe sowie zur Beseitigung oder zum Unschädlichmachen von Krankheitserregern oder Körperfremdstoffen Einsatz findet. Die Freisetzung der Substanz in der Implantatsumgebung hat einen positiven Effekt auf den Heilungsverlauf bzw. wirkt pathologischen Veränderungen des Gewebes infolge des chirurgischen Eingriffes entgegen. Diese pharmazeutisch aktiven Substanzen können dabei in der Vorrichtung aus dem oben genannten biodegradierbarem Polymermaterial integriert oder inkorporiert sein oder als Beschichtung beispielsweise in einer aktiven Beschichtung bestehend aus einem der soeben genannten Polymermaterialen auf der Vorrichtung vorliegen.

Als phamrazeutisch aktive Substanzen können beispielsweise antiproliferative, antiinflammatorische und/oder antimykotische Wirkstoffe aus der folgenden Liste gewählt werden:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Biolimus, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2w, Mitoxanthrone, Mycophenolatmofetil, c-myc-Antisense, [beta]-myc-Antisense, [beta]-Lapachon, Podophyllotoxin, Podophyllsäure-2-ethylhydrazid, Molgramostim (rhuGM-CSF), Peginterferon [alpha]-2b, Lanograstim (r-HuG-CSF), Macrogol, Selectin (Cytokinantagonist), Cytokininhibitoren, COX-2-Inhibitor, NFkB, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren wie Pentaerythrityltetranitrat und Syndnoeimine, S-Nitrosoderivate, Tamoxifen, Staurosporin, [beta]-Estradiol, [alpha]-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, TyrosinKinase-Inhibitoren (Tyrphostine), Paclitaxel und dessen Derivate wie 6-[alpha]-Hydroxy-Paclitaxel, Taxotere, Kohlensuboxids (MCS) und dessen macrocyclische Oligomere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, [beta]-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, PlasminogenAktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, E-noxoparin, desulfatiertes und N-reacetyliertes Heparin (Hemoparin<(R)>), Gewebe-Plasminogen-Aktivator, GpIIb/IIIa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon [alpha], [beta] und [gamma], Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol" Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

Vorzugsweise sind die Wirkstoffe aus den Klassen Glucocorticoide und/oder Aminoglykosidantibiotika, wie beispielsweise Dexamethason oder in eine Poly D,L Lactid - Beschichtung integriertes Gentamicin.

Gemäß eines zweiten Aspektes wird ein elektromedizinisches Implantat beschreiben, bei dem die implantierbare Vorrichtung in das Gehäuse integriert dies. Das elektromedizinische Implantat umfasst dabei neben der soeben beschriebenen Vorrichtung eine elektronische Schaltung mit Energieversorgung, ein mehrteiliges Gehäuse, welches die Schaltung und die Energieversorgung hermetisch dicht umschließt, und ein Anschlussgehäuse, welches am mehrteiligen, hermetisch dichten Gehäuse befestigt ist und welches Anschlüsse für mindestens Elektrodenleitung aufweist, wobei die Anschlüsse mit der elektronischen Schaltung elektrisch verbunden sind. Das elektromedizinische Implantat zeichnet sich dabei dadurch aus, dass die zweite Außenfläche der Vorrichtung einen Teil des Gehäuses bildet. Bevorzugt sind die erste Außenfläche und das mindestens eine Führungsmittel von außen zugänglich. Durch diese Ausgestaltung wird eine Schädigung der Gewebetasche noch weiter verhindert. Weiterhin vereint dieser Aspekt alle weiter oben beschriebenen Vorteile.

Im Folgenden wird der Gegenstand der Patentanmeldung anhand von Fig. beschrieben. Es zeigen:
- Fig. 1A bis Fig. 1C:: eine implantierbare Vorrichtung gemäß einer ersten Weiterbildung eines ersten und zweiten Aspektes, bei der die Führungsmittel um zwei imaginäre Achsen herum angeordnet sind
- Fig. 2A bis Fig. 2C:: eine implantierbare Vorrichtung gemäß einer zweiten Weiterbildung des zweiten Aspektes, bei der die Führungsmittel als Bahnen ausgelegt sind

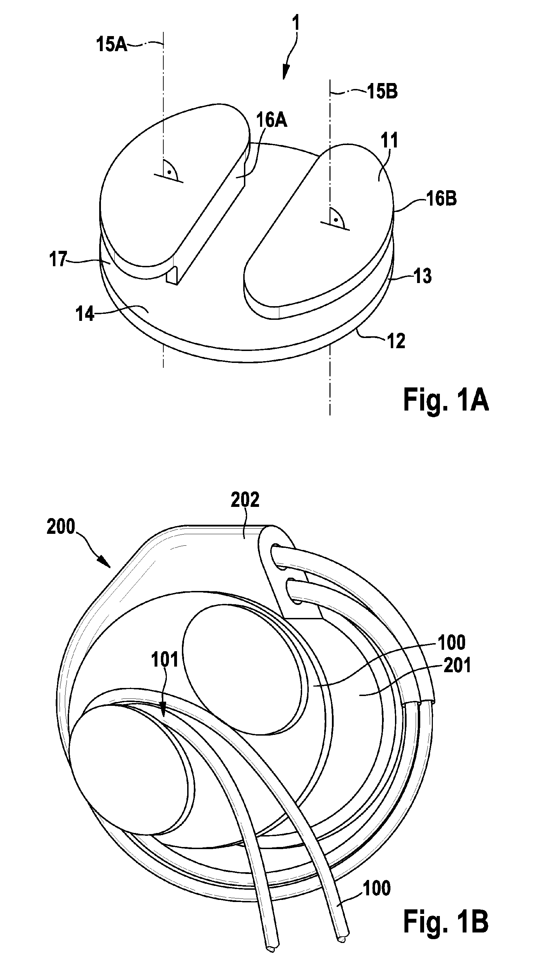

Fig. 1A bis Fig. 1C zeigen die implantierbare Vorrichtung 1 mit einer ersten Außenfläche 11 und einer zweiten, der ersten Außenfläche gegenüber liegenden und parallel verlaufenden Außenfläche 12. Letztere ist in dieser perspektivischen Darstellung nicht sichtbar. Die Vorrichtung wird durch eine umlaufende Außenkante 13 begrenzt.

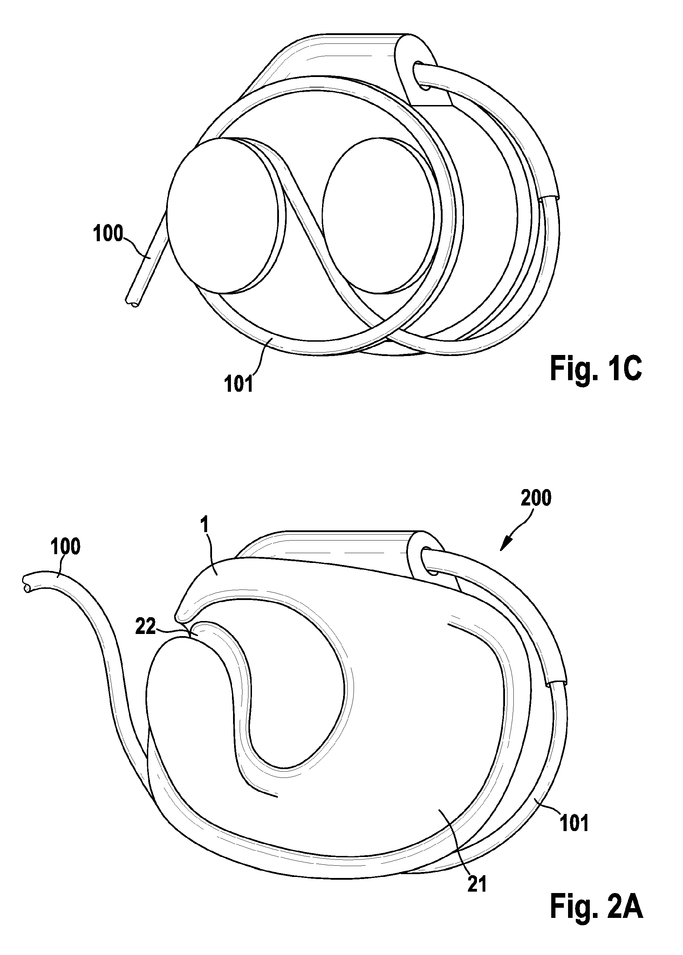

In die erste Außenfläche 11 ist ein Führungsmittel 14 integriert, in diesem Fall in die erste Außenfläche 11 eingelassen. Das bedeutet, dass das Führungsmittel ein Höhenniveau aufweist, das niedriger ist als die erste Außenfläche. In dieser Weiterbildung des ersten Aspektes verlaufen die Führungsmittel 14 um zwei imaginären Achsen 15A und 15B, so dass zwei zylinderartiges Gebilde 16A und 16B entsteh3n, um die - wie in Fig. 1B und 1C gezeigt - eine oder mehrere Elektrodenleitungsabschnitte 101 einer oder mehrerer Elektrodenleitungen 100 geführt werden können.

Wie exemplarisch dargestellt ist, ist die Höhe des zylindrischen Gebildes 16A oder 16B mindestens so hoch, dass der Elektrodenleitungsabschnitt 101 nicht oder kaum über das zylindrische Gebilde hinausragt. Das zylindrische Gebilde 16B zeigt eine Höhe, die in etwa dem Durchmesser des Elektrodenleitungsabschnittes 101 entspricht. Dagegen ist das zylindrische Gebilde 16A so hoch, dass eine seitliche Aussparung 17 gebildet werden kann. Diese Aussparung 17 hat dabei eine Höhe, die geringfügig höher als der Durchmesser des Elektrodenleitungsabschnittes 101 ist. So kann dieser Elektrodenleitungsabschnitt 101 in der Aussparung 17 geführt werden und verhindert so ein Verrutschen oder Lösen der Elektrode von der implantierbaren Vorrichtung. Die Tiefe der Aussparung 17 kann dabei so gewählt werden, dass zwei oder mehrere Elektrodenleitungen in ihr geführt werden können. Dies ist beispielsweise in Fig. 1B dargestellt.

Alternativ oder zusätzlich ist es auch denkbar, die Fixierung der Elektrodenleitungsabschnitte 101 an der implantierbaren Vorrichtung 1 mittels eines Fixierungsmittels zu verwirklichen. Dazu kann beispielsweise biokompatible Knetmasse, d.h. biokompatible Polymere (beispielsweise die weiter oben Genannten) mit Erweichungstemperaturen von 37°C oder darunter (die beispielsweise im Granulierungsprozess vor einer Extrusion eingestellt bzw. provoziert werden kann) oder thermoplastisch verformbares Silikon genutzt werden. Ebenfalls ist es beispielsweise möglich, eine Folie oder ein sehr dünnes Blättchen von oben auf der ersten Außenfläche 11 zu befestigen, beispielsweise ebenso reversibel wie soeben beschrieben. Diese Abdeckung kann auch die Außenkante 13 bedecken. So ist der komplette Elektrodenleitungsabschnitt 101 umschlossen und gegen Einwachsen geschützt.

Die implantierbare Vorrichtung kann durch ihre flache Bauweise in die Gewebetasche mit dem elektromedizinischen Implantat gelegt werden. Fig. 1B zeigt aber auch, dass die implantierbare Vorrichtung 1 der soeben beschriebenen Art an einem elektromedizinischen Implantat 200 angebracht werden kann. Das Implantat 200 besteht aus einem Gehäuse 201, das in der Regel zwei- oder mehrteilig ist und aus leitendem Material wie Titan besteht, und einem Anschlussgehäuse 202, welches aus einem nicht leitenden Kunststoff besteht. innerhalb des Anschlussgehäuses befinden sich Steckeraufnahmen, um die am proximalen Ende der Elektrodenleitung befindlichen Stecker aufnehmen zu können. Die Steckeraufnahmen umfassen Kontaktflächen, welche auch mit einer elektronischen, von einer Energieversorgung gespeisten, Schaltung verbunden sind. Die implantierbare Vorrichtung 1 kann an der zweiten Außenfläche 12 mit dem Gehäuse 201 des elektromedizinischen Implantats 200 verbunden sein. Dabei ist es wichtig, die Verbindung lösbar zu gestalten, damit es möglich ist, in einfacher Weise das elektromedizinische Implantat auszutauschen, wenn dieses "end of life" erreicht hat. Diese Verbindung kann beispielsweise durch lösbare Klebstoffe erreicht werden. Der Arzt kann dann also zum Austausch des elektromedizinischen Implantats 200 die Gewebstasche eröffnen, die Stecker der Elektrodenleitungen 100 aus den Steckeraufnahmen entfernen, die implantierbare Vorrichtung 1 vom Gehäuse 201 des elektromedizinischen Implantates lösen, das neue elektromedizinische Implantat einsetzen, die implantierbare Vorrichtung wieder am Gehäuse des neuen elektromedizinischen Implantates befestigen und den elektrischen Kontakt zur Elektrodenleitung wieder herstellen, indem die Stecker in die Steckeraufnahmen eingeführt werden.

Gemäß eines zweiten Aspekts kann die implantierbare Vorrichtung wie in Fig. 1C gezeigt auch in das Gehäuse 201 des elektromedizinischen Implantats integriert sein. Dazu bildet die zweite Außenfläche das ein Teil (beispielsweise eine Halbschale) des zwei- oder mehrteiligen Gehäuses. In diesem Fall ist es notwendig, die Elektrodenleitungsabschnitte 101 mit zusätzlichen Fixierungsmitteln komplett zu bedecken, damit kein Einwachsen der Elektrodenleitung 100 in die Gewebstasche vermieden wird. Dann kann die Elektrodenleitung leicht freipräpariert werden, ohne dass sie beschädigt wird.

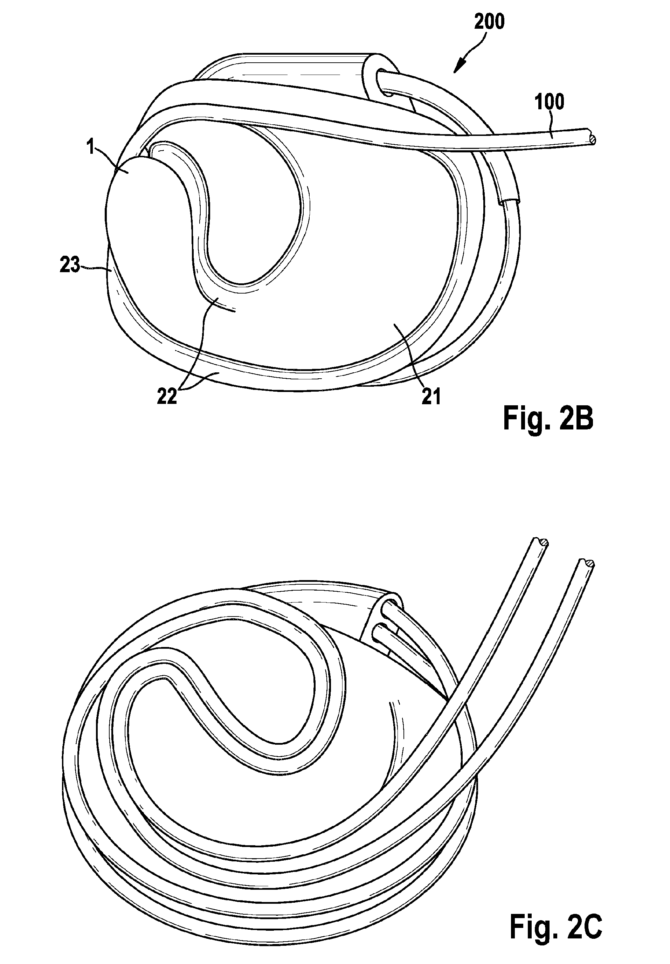

Fig. 2A bis Fig. 2C zeigen die implantierbare Vorrichtung 1 in einer zweiten Weiterbildung, in diesem Fall als integraler Bestandteil des Gehäuses 201 des elektromedizinischen Implantates 200. Das Führungsmittel ist in diesem Fall eine Bahn 22, welche in der ersten Außenfläche 21 eingelassen ist. Diese Bahn 22 kann dabei auch nur teilweise von der Außenfläche umgeben sein, so dass der Elektrodenleitungsabschnitt 101 an der Außenkontur des elektromedizinischen Implantats entlang geführt werden kann, wenn die Außenkante 23 der implantierbaren Vorrichtung 1 an die Außenkontur angepasst ist. Die Tiefe der Bahnen 23 ist dabei ebenso wie im vorher beschriebenen Ausführungsbeispiels so gewählt, dass die Elektrodenleitungsabschnitte kaum über die erste Außenfläche herausragt. Ebenso können die Bahnen 23 für eine oder mehrere Elektrodenleitungsabschnitte ausgelegt sein.

Auch in diesen Ausführungsformen können sämtliche weiter oben beschriebenen zusätzlichen Fixierungs- oder Schutzmittel zum Einsatz kommen.

Denkbar ist auch eine Kombination aus beiden Weiterbildungen der implantierbaren Vorrichtung. Beispielsweise ist eine solche Kombination in Fig. 1C gezeigt. Dort verläuft ein Teil des Elektrodenleitungsabschnittes 101 in einer Bahn 22, um so an der Stelle, an dem zwei Teile des Elektrodenleitungsabschnittes 101 übereinander liegen, keinen zu großen Materialauftrag zu generieren, welcher dann über die erste Außenfläche 11 herausragt. Diese Maßnahme kann auch bei der zweiten Ausführung gemäß Fig. 1A bis 1C erfolgen.

## Patentansprüche

1. Implantierbare Vorrichtung zur reversiblen Aufnahme eines Elektrodenleitungsabschnittes mindestens einer Elektrodenleitung, umfassend
- eine erste Außenfläche, und
- eine zweite Außenfläche, die sich beabstandet parallel zur ersten Außenfläche
erstreckt,
wobei die erste Außenfläche mindestens ein Führungsmittel umfasst, mit dessen Hilfe der Elektrodenleitungsabschnitt geführt und aufgenommen werden kann.

2. Implantierbare Vorrichtung nach Anspruch 1, wobei das mindestens eine Führungsmittel in die erste Außenfläche integriert, bevorzugt so eingelassen oder vertieft ist, dass der mindestens eine Elektrodenleitungsabschnitt nicht die erste Außenfläche überragt.

3. Implantierbare Vorrichtung nach Anspruch 2, wobei das mindestens eine Führungsmittel um eine Achse herum angelegt ist, welche eine Normale zur ersten Außenfläche ist, so dass der mindestens eine Elektrodenleitungsabschnitt im Führungsmittel um die Achse herum geführt werden kann.

4. Implantierbare Vorrichtung nach Anspruch 3, wobei das mindestens eine Führungsmittel so geformt ist, dass es die erste Außenfläche kreis-, gleichdick-, oval-oder achteckförmig mit abgerundeten Ecken umschließt, so dass ein zylinderartiges Gebilde entsteht.

5. Implantierbare Vorrichtung nach einem der Ansprüche 3 oder 4, wobei das Führungsmittel Aushöhlungen umfasst, die die erste Außenfläche zumindest teilweise seitlich unterhöhlen, um so ein Verrutschen des mindestens einen Elektrodenleitungsabschnittes zu verhindern.

6. Implantierbare Vorrichtung nach Anspruch 2, wobei das mindestens eine Führungsmittel als vorgeformte Bahn in die erste Außenfläche integriert, bevorzugt eingelassen oder vertieft ist, in welcher der mindestens eine Elektrodenleitungsabschnitt aufgenommen werden kann.

7. Implantierbare Vorrichtung nach Anspruch 6, wobei die vorgeformte Bahn so gestaltet und in die erste Außenfläche integriert, bevorzugt eingelassen oder vertieft ist, dass sie an jeder Stelle der Außenfläche nur einmal geführt wird, um zu verhindern, dass die mindestens einen Elektrodenleitungsabschnitte übereinander liegen.

8. Implantierbare Vorrichtung nach Anspruch 6, wobei die vorgeformte Bahn so gestaltet und in die erste Außenfläche integriert, bevorzugt eingelassen oder vertieft ist, dass die Bahn Aussparungen an Stellen aufweist, an denen der mindestens eine Elektrodenleitungsabschnitt mehrfach übereinander verläuft, um zu verhindern, dass der mindestens eine Elektrodenleitungsabschnitt nicht die erste Außenfläche überragt.

9. Implantierbare Vorrichtung nach einem der Ansprüche 1 bis 8 zusätzlich umfassend eine umlaufende Außenkante, die die Vorrichtung begrenzt, wobei die umlaufende Außenkante an die Außenkontur eines elektromedizinisches Implantats angepasst ist.

10. Implantierbare Vorrichtung nach einem der Ansprüche 1 bis 9, welches an der zweiten Außenfläche Mittel aufweist, mit welchen die Vorrichtung an ein elektromedizinisches Implantat reversibel anbringbar ist.

11. Implantierbare Vorrichtung nach einem der Ansprüche 1 bis 10, welches aus einem harten, nicht formbaren Material oder weichen, verformbaren Material gefertigt ist, das vorzugsweise biodegradierbar oder bioresorbierbar ist, wobei das biodegradierbare oder bioresorbierbare Material besonders bevorzugt eine biodegradierbare Metalllegierung oder ein biodegradierbares Polymer ist.

12. Implantierbare Vorrichtung nach einem der Ansprüche 1 bis 11, wobei die Vorrichtung mit einer oder mehreren pharmazeutisch aktiven Substanzen versehen ist.

13. Elektromedizinisches Implantat, umfassend eine Vorrichtung nach einem der Ansprüche 1 bis 12, umfassend:
- eine elektronische Schaltung mit Energieversorgung,
- ein mehrteiliges Gehäuse, welches die Schaltung und die Energieversorgung hermetisch dicht umschließt, und
- ein Anschlussgehäuse, welches am mehrteiligen, hermetisch dichten Gehäuse befestigt ist und welches Anschlüsse für mindestens Elektrodenleitung aufweist, wobei die Anschlüsse mit der elektronischen Schaltung elektrisch verbunden sind
**dadurch gekennzeichnet, dass**
die zweite Außenfläche der Vorrichtung einen Teil des Gehäuses bildet.

14. Elektromedizinisches Implantat nach Anspruch 13, **dadurch gekennzeichnet, dass** die die erste Außenfläche und das mindestens eine Führungsmittel von außen zugänglich sind.
